# EUROPEAN PATENT SPECIFICATION

(11) **EP 1 213 280 B1**
(45) Date of publication and mention of the grant of the patent: **29.09.2004**
(21) Application number: 01309780.3
(22) Date of filing: 21.11.2001
(51) Int. Cl.: C07C 291/02, C07C 291/04, B01J 21/16

(54) **A process for the preparation of amine oxides**
Verfahren zur Herstellung von Aminoxiden
Procédé de préparation d'oxydes d'amines

(30) Priority: 22.11.2000 US 721585; 30.11.2000 EP 00310609
(43) Date of publication of application: 12.06.2002
(73) Proprietor: Council of Scientific and Industrial Research, New Delhi 110 001 (IN)
(72) Inventor: Choudary, B., Indian Institute of Chemical Tech., 500 007 Andhra Pradesh (IN); Bharathi, B., Indian Institute of Chemical Tech., 500 007 Andhra Pradesh (IN); Kantam, M., Indian Institute of Chemical Tech., 500 007 Andhra Pradesh (IN); Reddy, C., Indian Institute of Chemical Tech., 500 007 Andhra Pradesh (IN); Raghavan, K. Indian Institute of Chemical Tech., 500 007 Andhra Pradesh (IN)
(74) Representative: Beresford, Keith Denis Lewis

(56) References cited:
- US-A- 3 274 252
- US-A- 4 596 874
- US-A- 4 774 212
- US-A- 6 124 506
- VENKATACHALAPATHY C ET AL: "A CLEAN CLAY CATALYSED SYNTHESIS OF A,N-DIARYLNITRONES" SYNTHETIC COMMUNICATIONS, MARCEL DEKKER, INC., BASEL, CH, vol. 27, no. 23, 1997, pages 4041-4047, XP000987142 ISSN: 0039-7911

## Description

### Field of the invention:

The present invention relates to an improved process for the preparation of high quality amine oxides from secondary and tertiary aliphatic amines. More particularly, the present invention relates to an improved process for the preparation of amine oxides from secondary and tertiary aliphatic amines useful in the preparation of hair conditioners and shampoos, toothpaste, laundry detergent powder, fabric softeners, toilet soap bars and cosmetics, surfactants as well as in other applications as synthetic intermediates and excellent spin trapping reagents.

### Background of the invention:

The N-oxides holds a key position in the chemistry of heterocycles as well as in biomedical area. The tertiary amine oxides are widely used in treatment of fabrics and preparation of hair conditioners and shampoos, toothpaste, laundry detergent powder, fabric softeners, toilet soap bars and cosmetics as well as in other applications. They were also used as stoichiometric oxidants in metal catalysed hydroxylation and epoxidation reactions of olefins. On the other hand, the oxides derived from secondary amines, called nitrones are highly valuable synthetic intermediates and excellent spin trapping reagents. In particular nitrones are excellent 1,3 dipoles and have been utilized for the synthesis of various nitrogen containing biologically active compounds e.g. alkaloids and lactams.

Conventionally tertiary amine oxides are prepared by oxidation of respective tertiary amines with strong oxidising agent like aqueous hydrogen peroxide in a solvent such as water, lower alcohol, acetone or acetic acid. A dilute or preferably concentrated (30-90% by weight) hydrogen peroxide solution is added in stoichiometric or greater amount to an aqueous solution containing the tertiary amine to obtain amine oxide, (U.S. Pat No. 3,215, 741). The drawback is that the reaction transforms into a gel resembling a thick paste long before completion of the reaction, which retards further reaction. The yields are only 30-40% by weight of amine oxide. Later several methods such as incorporation of catalyst and/chelating agent have been developed in order to increase the quality and yields of the product.

In case of secondary amines, the classical methods involve the condensation of N-monosubstituted hydroxylamines with carbonyl compounds or the direct oxidation of N,N-disubstituted hydroxylamines. Later direct oxidation of secondary amines using several oxidising systems such as R₂C(µ-O₂), Na₂WO₄-H₂O₂, SeO₂, TPAP-NMO and UHP-M (M=Mo, W), MTO-H₂O₂ have been developed to accomplish nitrones under homogenous conditions. The drawback in all the above cases is the difficulty in recovering the homogeneous catalyst/reagents from the reaction mixture.

Reference may be made to a U.S. patent 3,283,007 wherein the oxidation of tertiary amines using diethylene triamine penta/tetra acetic acid as chelating agent and sometimes contaminated with heavy metals is recommended to improve the yield. The hydrogen peroxide solution employed has concentration of at least 30-75% by weight. The disadvantages of this process are high reaction temperatures ranging between 40-100 °C, longer reaction periods, and lower yields of amine oxides.

Reference may be made to US patent 3,424,780, wherein high yields of tertiary amine oxides are achieved by carrying out the oxidation of tertiary amine with 30-70% by weight of aqueous hydrogen peroxide using 0.01 to 2% weight of carbon dioxide, in presence of a chelating agent, tetra acetylene diamine, a salt thereof, polyphosphates, stannates, a hydroxy carboxylic acid salts or the salt of poly carboxylic acid. The reaction is carried out at a temperature ranging from 40 to 80°C. The disadvantages of this process are high reaction temperature, longer reaction periods and the amine oxide formed is intensively coloured when carbon dioxide atmosphere is used to speed up the reaction and this method necessitates injecting a gas which requires handling facilities. Another disadvantage is more than 30% by weight of hydrogen peroxide is not environmentally friendly.

Reference may be made to another US patent 4,889,954 wherein the tertiary amines are reacted in high yields to give the corresponding amine oxides with a low content of nitrosamine, the oxidation of tertiary amine being carried out in the presence of a dialkyl carboxylic acid ester as catalyst and if appropriate, ascorbic acid as a co-catalyst using 45-70% by weight of hydrogen peroxide. The drawbacks in the above process are the requirement of frequent addition of water to avoid gel formation, high reaction temperatures, longer reaction periods and difficulty in separation of the catalyst from the reaction mixture.

Reference may be made to another US patent 4,565,891 wherein octacyano molybdate or iron salts are used as catalysts and molecular oxygen for oxidation of tertiary amines at high pressures and temperatures. The main drawback of this process is the need of very high temperature of 90-130°C and very low yields of amine oxide reporting 11-52% of conversion.

Reference may be made to a US patent 5,130,488 wherein the solid amine oxide can be prepared by reacting a tertiary amine with hydrogen peroxide using carbon dioxide in presence of acetate and cooling to precipitate the product. This process is superior to previously known methods of preparing amine oxides. However, its use can sometimes lead to cleavage of the solvents, plating on the walls of the vessel used for the precipitation, contamination of the product with residual peroxide, and or discoloration of the product.

Reference may be made to a publication by Walter W. Zajac et al., J. Org. Chem.; 53, 5856, 1988 wherein the oxidation of secondary and tertiary amines using 2-sulfonyloxyxaziridines (Davis Reagents) was reported. The drawback of the above process is, the reagent was used in stoichiometric amounts.

Reference may be made to a publication by Shun-Ichi Murahashi *et al.,* J. Org. Chem.; 55, 1736, 1990 wherein the sodium tungstate was used as catalyst for the oxidation of secondary amines. The drawback is the difficulty in recovery of the catalyst from homogeneous conditions.

Another reference may be made to publication by Murray *et al.,* J. Org. Chem.; 61, 8099, 1996 wherein methyltrioxorhenium was used as a catalyst in oxidation of secondary amines. The drawback is the difficulty in recovery of the catalyst.

United States Patent No. 4,596,874 discloses synthesis of an amine oxide from a secondary amine using peroxide in the presence of a catalyst selected from tungsten compounds, molybdenum compounds, vanadium compounds, titanium compounds, palladium compounds, rhodium compounds, ruthenium compounds and nickel compounds. Best results are reported with a hydrogen peroxide-sodium tungstate system.

United States Patent No. 6,124,506 discloses a process for making glycol ethers by reacting olefin oxide with alcohol using a layered double hydroxide clay as catalyst.

### Objects of the invention

The main object of the present invention is to provide an eco-friendly and simple process for N-oxidation of secondary and tertiary amines using layered double hydroxides exchanged with anions of transition metal oxides as a catalyst, which is cheaper, noncorrosive and recyclable catalyst utilising only lower percentage of hydrogen peroxide at room temperatures to give high yields of product.

Another object of the present invention is to provide an improved process for the preparation of tertiary amine oxides and secondary amine oxides (nitrones), widely used in detergents, shampoos, fabric softers and biomedical area.

A further object of the invention is to provide an environmentally friendly process for the preparation of tertiary amine oxides and nitrones, using water alone or in combination with dodecylbenzenesulfonic acid sodium salt as an additive.

### Summary of the Invention

Accordingly, the present invention provides an improved process for the preparation of amine oxides of a very high quality which comprises reacting tertiary and secondary amines with hydrogen peroxide as an oxidant in presence of a recyclable heterogeneous catalyst, layered double hydroxides exchanged with anion of transition metal oxides, with tungstate, molybdate, vanadate, and their polyanions i.e. polyoxometallates, in a solvent selected from water or water containing docecylbenzenesulfonic acid sodium salt as additive at a temperature range between 10-25°C for a period of 1-6 hours under continuous stirring and separating the product by simple filtration and subsequent evaporation of solvents by known methods.

In an embodiment of the present invention, the heterogeneous catalyst used is the layered double hydroxide exchanged with transition metal oxides selected from a group consisting of tungstate, molybdate, vanadate and their polyanions i.e. polyoxometalates, having formula I: [M^{II}₍₁₋ₓ₎M^{III}ₓ(OH)₂][Mⁿ⁻]_{x/2}.zH₂O, which is derived from LDH having formula II [M^{II}₍₁₋ₓ₎M^{III}ₓ(OH)₂][Aⁿ⁻ ]_{x/2}.zH₂O where Mⁿ⁻ is an anion of transition metal oxide selected from the group consisting of W, Mo, V and Aⁿ⁻ is an interstitial anion, selected from nitrate and chloride and M^{II} is a divalent cation selected from the group consisting of Mg²⁺, Mn²⁺, Fe²⁺, V²⁺, Co²⁺, Ni²⁺, Cu²⁺, Zn²⁺, Pd²⁺, or Ca²⁺, and M^{III} is a trivalent ion selected from the group consisting of Al³⁺, Cr³⁺, V³⁺, Mn³⁺, Fe³⁺, Co³⁺, Ni³⁺, Rh³⁺, Ru³⁺, Ga³⁺ or La³⁺, x is the mole fraction having a value ranging from 0.2 to 0.33 and Z is the number of water molecules ranging from 1 to 4.

In another embodiment of the present invention, the tertiary amines used have the general formula R¹R²NR³ wherein R¹, R², and R³, which may be the same or different, are straight-chain or branched-chain groups selected from alkyl, alkenyl and aralkyls having C₁-C₂₄ carbons including dimethyl decyl amine, dimethyl dodecyl amine, dimethylbenzylamine, triethylamine, tributylamine and cyclic amines selected from imidazolines, pyridines, N-substituted piperazines, N-substituted piperadines or N-substituted morpholines, e.g., N-methylmorpholine.

In another embodiment of the present invention, the secondary amines used have general formula R¹R²NH wherein R¹ and R² may be the same or different and are straight-chain or branched-chain groups selected from alkyl, alkenyl and aralkyl : having C₁-C₂₄ carbons, including dibutyl amine, dibenzyl amine, N-benzyl phenethylamine, N-phenyl benzylamine and cyclic amines selected from piperidine, 1,2,3,4, tetrahydro isoquinoline.

In another embodiment, of the present invention aqueous hydrogen peroxide is added slowly in a controlled manner for a period up to 120 min.

In yet another embodiment of the present invention, the catalyst introduced in the system is 6-12% by weight of anion of transition metal oxides selected from tungstate, molybdate, vanadate and their polyanions as polyoxometalates.

In still another embodiment of the present invention, the amount of hydrogen peroxide used is 2 to 6 moles per mole of tertiary or secondary amine.

### Detailed Description of the Invention:

The catalyst used in the invention comprises a recyclable heterogeneous catalyst. i.e. layered double hydroxides exchanged with tungstate, molybdate, vanadate and their polyanions i.e. polyoxometalates that catalyse oxidation of secondary and tertiary amines in water or in water with dodecylbenzenesulfonic acid sodium salt as an additive. The advantages such as low cost of the catalyst, reusability for several times and its ability to oxidise the amines at 10-25 °C, below or at room temperature in a shorter period make the present invention a promising candidate for a clean and efficient industrial route to amine oxide preparation.

Co-pending European Application No 00310609.3 discloses a process for the preparation of amine oxides of high quality by reacting tertiary and secondary amines with hydrogen peroxide as an oxidant in presence of a recyclable heterogeneous catalyst, layered double hydroxides exchanged with anion of transition metal oxides, with tungstate, molybdate, vanadate, and their polyanions i.e. polyoxometallates, in an organic solvent at a temperature ranges between 10-25°C for a period of 1-6 hours under continuous stirring and separating the product by simple filtration and subsequently evaporation of solvents by known methods.

The heterogeneous catalyst used is the layered double hydroxide exchanged with transition metal oxides selected from a group consisting of tungstate, molybdate, vanadate and their polyanions i.e. polyoxometalates, having formula I: [M^{II}₍₁₋ₓ₎M^{III}ₓ(OH)ₓ][Mⁿ⁻ ]ₓ₋₂.zH₂O, which is derived from LDH having formula II [M^{II}₍₁₋ₓ)M^{III}ₓ(OH)₂][Aⁿ⁻]ₓ₋₂.zH₂O where Mⁿ⁻ is an anion of transition metal oxide, selected from the group consisting of W, Mo, V and Aⁿ⁻ is interstitial anion, selected from nitrate, chloride and M^{II} is a divalent cation selected from the group consisting of Mg²⁺, Mn²⁺, Fe²⁺, V²⁺, Co²⁺, Ni²⁺, Cu²⁺, Zn²⁺ Pd²⁺, or Ca²⁺ and M^{III} is a trivalent ion selected from the group consisting of Al³⁺, Cr³⁺, V³⁺, Mn³⁺, Fe³⁺, Co³⁺, Ni³⁺, Rh³⁺, Ru³⁺, Ga³⁺ or La³⁺, x is the mole fraction having a value ranging from 0.2 to 0.33 and z is the number of water molecules ranging from 1 to 4.

The tertiary amines used have the general formula R¹R²NR³ wherein R¹, R² and R³, which may be the same or different, are straight-chain or branched-chain groups selected from alkyl, alkenyl and aralkyl having C₁-C₂₄ carbons such as dimethyl decyl amine, dimethyl dodecyl amine, dimethylbenzylamine, triethylamine, tributylamine and cyclic amines selected from imidazolines, pyridines, N-substituted piperazines, N-substituted piperadines or N-substituted morpholines, e.g., N-methylmorpholine. The secondary amines used have general formula R¹R²NH wherein R¹ and R² may be the same or different and are straight-chain or branched-chain groups selected from alkyl, alkenyl and aralkyl having C₁-C₂₄ carbons, such as dibutyl amine, dibenzyl amine, N-benzyl phenethylamine, N-phenyl benzylamine and cyclic amines selected from piperidine, 1,2,3,4, tetrahydro isoquinoline.

Aqueous hydrogen peroxide is added slowly in a controlled manner for a period up to 120min. The catalyst introduced in the system is 6-12% by weight of anion of transition metal oxides selected from tungstate, molybdate, vanadate and their polyanions as polyoxometalates. The amount of hydrogen peroxide used is 2 to 6 moles per mole of amine.

### Scientific explanation:

The catalycic cycle in the oxidation of amines to amine oxides involves the easy formation of peroxotungstate, HOOWO₃⁻/HOOWO₆⁻ on interaction of tungstate with hydrogen peroxide. These peroxy species will act as an active species for the oxidation of secondary/tertiary amines as described by Murahashi *et*.*al*., for the Na₂WO₄ catalysed oxidation of secondary amines by hydrogen peroxide. The secondary amine undergoes nucleophilic reaction with peroxotungstate species to give hydroxylamine. Further oxidation of hydroxylamine followed by dehydration gives nitrone. In case of tertiary amines, the oxygen transfer occurs from peroxotungstate species to tertiary amine in a single step to form tertiary amine oxide. The species HOWO₃⁻/HOWO₆⁻ thus formed is readily oxidized with another molecule of H₂O₂ to give peroxo tungstate HOOWO₃⁻/HOOWO₆⁻, thus completing the catalytic cycle.

The following examples are given by way of illustration of the present invention and therefore should not be construed to limit the scope of the present invention.

### Reference Example 1

### Preparation of the various catalysts

### 1. Preparation of Mg-Al hydrotalcite (LDH) chloride:

Mg-Al-Cl hydrotalcite (3:1) is prepared as follows: About 200ml of decarbonated and deionised water was taken into a 1 litre four necked round bottomed tlask and stirred at 25 °C with a magnetic stirrer under a nitrogen flow. The mixture (Al³⁺=0.05mol/l), (Mg²⁺= 0.15mol/l) of decarbonated solution of AlCl₃.9H₂O (12.07g), MgCl₂.6H₂O (30.49g) (obtained from M/s. Fluka, a Sigma Aldrich Company, Switzerland) and aqueous solution of sodium hydroxide (16g, 0.2mol/l) were added continuously drop-wise from a burette, the pH of the reaction mixture being kept at 10.00-10.2 during the reaction. The precipitate obtained was filtered, washed with deionised and decarbonated water and dried at 70°C for 15h.

### a) Preparation of Mg-Al hydrotalcite (LDH) tungstate (Catalyst A):

To reach anion exchange of degree of 12%, 1 g of Mg-Al-Cl hydrotalcite was stirred in 100ml of aqueous solution of 1.87mM (0.616g) sodium tungstate (obtained from M/s. Fluka, a Sigma Aldrich Company, Switzerland), at 293K for 24h. The solid catalyst was filtered, washed with deionised and decarbonated water and lyophilized to dryness.

### b) Preparation of Mg-Al hydrotalcite (LDH) molybdate (Catalyst B):

To reach anion exchange of degree of 12%, 1 g of Mg-Al-Cl hydrotalcite was stirred in 100ml of aqueous solution of 1.87mM (0.452g) sodium molybdate (obtained from M/s. Fluka, a Sigma Aldrich Company, Switzerland), at 293K for 24h. The solid catalyst was filtered, washed with deionised and decarbonated water and lyophilized to dryness.

### c) Preparation of Mg-Al hydrotalcite (LDH) vanadate (Catalyst C):

To reach anion exchange of degree of 12%, 1 g of Mg-Al-Cl hydrotalcite is stirred in 100ml of aqueous solution of 1.87mM (0.456g) sodium vanadate (obtained from M/s. Fluka, a Sigma Aldrich Company, Switzerland), at 293K for 24h. The solid catalyst was filtered, washed with deionised and decarbonated water and lyophilized to dryness.

### 2. Preparation of Mg-Al hydrotalcite (LDH) nitrate:

Magnesium nitrate hexahydrate (30.8g, 0.12mol) and aluminium nitrate nonahydrate (15.0g, 0.04mol) were dissolved in 100ml of deionised and decarbonated water. The pH of the solution was adjusted to 10 by adding 2M NaOH. The resulting suspension was stirred for 2h at room temperature. The precipitate hydrotalcite was collected by filtration under N₂ atmosphere and dried overnight at 80 °C.

### a) Preparation of Mg-Al hydrotalcite (LDH) tungstate (Catalyst D):

To reach anion exchange of degree of 12%, 1 g of Mg-Al-NO₃ hydrotalcite was stirred in 100ml of aqueous 1.87mM (0.616g) sodium tungustate (obtained from M/s. Fluka, a Sigma Aldrich Company, Switzerland), at 293K for 24h. The solid catalyst was filtered, washed with deionised and decarbonated water and lyophilized to dryness.

### 3. Preparation of Mg-Al hydrotalcite (LDH) carbonate:

Mg-Al-CO₃ hydrotalcite (3:1) is prepared as follows: An aqueous solution (0.280 l) containing Mg(NO₃)₂.6H₂O (0.2808 mol) and Al(NO₃)₃.9H₂O (0.093 mol) (obtained from M/s. Fluka, a Sigma Aldrich Company, Switzerland) was added slowly to a second solution (0.280 l) containing NaOH (0.6562 mol) and Na₂CO₃ (0.3368 mol) in a 1.0 l round bottomed flask under vigorous stirring. The addition took nearly 3h. Then the slurry was heated to 338 K for 16h. The precipitate formed was filtered off and washed with hot distilled water until the pH of the filtrate was 7. The precipitate was dried in an oven at 353 K for 15h.

### a) Preparation of Mg-Al hydrotalcite (LDH) tungstate (Catalyst E):

To reach anion exchange of degree of 12%, 1 g of Mg-Al- CO₃ calcined (at 723 K for 6h in a flow of air) hydrotalcite was stirred in 100ml of aqueous solution of 1.87mM (0.616g) sodium tungstate (obtained from M/s. Fluka, a Sigma Aldrich Company, Switzerland), at 293K for 24h. The solid catalyst was filtered, washed with deionised and decarbonated water and lyophilized to dryness.

### 4.Preparation of Ni-Al hydrotalcite (LDH) chloride:

Ni-Al hydrotalcite chloride (3:1) was prepared as follows: About 200ml of decarbonated and deionised water was taken into a 1 litre four necked round bottomed flask and stirred at 25 °C with a magnetic stirrer under nitrogen flow. A mixture (Al³⁻ = 0.05mol/l), (Ni²⁻ = 0.15mol/l) of decarbonated solution of AlCl₃.9H₂O (12.07g), NiCl₂.6H₂O (35.65g) (obtained from M/s. Fluka, a Sigma Aldrich Company, Switzerland) and aqueous solution of sodium hydroxide (16g, 0.2mol/l) were added continuously drop-wise from a burette, the pH of the reaction mixture being kept at 10.00-10.2 during the reaction. The precipitate obtained was filtered, washed with deionised and decarbonated water and dried at 70 °C for 15h.

### a) Preparation of Ni-Al hydrotalcite (LDH) tungstate (Catalyst F):

To reach anion exchange of degree of 12%, 1 g of Ni-Al hydrotalcite chloride was stirred in 100ml of aqueous 1.87mM (0.616g) sodium tungstate (obtained from M/s. Fluka, a Sigma Aldrich Company, Switzerland), at room temperature for 24h. The solid catalyst was filtered, washed with deionised and decarbonated water and lyophilized to dryness.

### 5. Preparation of Ni-Al hydrotalcite (LDH) nitrate:

Nickel nitrate hexahydrate (34.8g, 0.12mol) and aluminium nitrate nonahydrate (15.0g, 0.04mol) were dissolved in 100ml of deionised and decarbonated water. The pH of the solution was adjusted to 10 by adding 2M NaOH. The resulting suspension was stirred for 2h at room temperature. The precipitate hydrotalcite was collected by filtration under N₂ atmosphere and dried overnight at 80 °C.

### a) Preparation of Ni-Al hydrotalcite (LDH) tungstate (Catalyst G):

To reach anion exchange of degree of 12%, 1 g of Ni-Al-NO₃ hydrotalcite was stirred in 100ml of aqueous 1.87mM (0.616g) sodium tungustate (obtained from M/s. Fluka, a Sigma Aldrich Company, Switzerland), at 293K for 24h. The solid catalyst was filtered, washed with deionised and decarbonated water and lyophilized to dryness.

### 6. Preparation of Bu₄N)₃PO₄[WO(O₂)₂]₄:

Hydrogen peroxide (30% w/w) 100mmol (10ml) was added to a solution of H₃[PW₁₂O₄₀] 6 mmol of tungsten (or 1.65g) in 1 ml of water. After 30 min an aqueous solution of tetrabutylammonium chloride (1.6 mmol) was slowly added. The resulting white precipitate was filtered out and washed with several times with water then air-dried.

### a)Preparation of LDH-{[PO₄WO(O₂)]₄}(Catalyst H):

The above synthesized complex (0.46 mmol) was exchanged on 1.0g of Mg-Al-Cl LDH in 3 ml of water, to this 1 ml of hydrogen peroxide (30%, w/w) was added drop-wise and stirring was continued for 16h at room temperature. Finally the catalyst was filtered and washed with water and water-acetone (1:1), acetone and dried on vacuum.

### Example 2

### Oxidation of N-methylmorpholine catalysed by tungstate exchanged with Mg/Al (3:1) layered double hydroxides using aqueous hydrogen peroxide

The four-necked flask was charged with 0.22ml (2mmol) of N-methylmorpholine, 200mg of ***catalyst A*** and 50ml of water. To the mixture was added dropwise 6.6ml (6 mmol) of a 30% by weight of aqueous solution of hydrogen peroxide for period of 0.5 hours in 2 to 3 portions at 25°C under continuous stirring. The reaction continued for another 0.5hour. After the completion of the reaction (followed by TLC), the catalyst was filtered off and washed with methanol. To the filtrate a small amount of manganese dioxide was added to decompose the unreacted hydrogen peroxide. The treated reaction mixture was filtered to remove the solid MnO₂ and concentrated under reduced pressure to obtain the product. The product thus obtained was purified by column chromatography to afford the corresponding amine oxide. N-methylmorpholine N-oxide of 96% yield was obtained. This product is commercially available from Fluka, Aldrich, Lancaster and Merck companies.

### Example 3

### Oxidation of N-methylmorpholine catalysed by tungstate exchanged with Mg/Al (3:1) layered double hydroxides using aqueous hydrogen peroxide: recycle-I

The oxidation reaction of N-methylmorpholine by using ***catalyst A*** which had been used in example 2 was performed in an identical procedure as detailed in example 2, without further addition of fresh catalyst. N-methylmorpholine N-oxide of 96% yield was obtained.

### Example 4

### Oxidation of N-methylmorpholine catalysed by tungstate exchanged with Mg/Al (3:1) layered double hydroxides using aqueous hydrogen peroxide: recycle-II

The oxidation reaction of N-methylmorpholine by using ***catalyst A*** which had been used in example 3 was performed in an identical procedure as detailed in example 2, without further addition of fresh catalyst. N-methylmorpholine N-oxide of 95% yield was obtained.

### Example 5

### Oxidation of N-methylmorpholine catalysed by tungstate exchanged with Mg/Al (3:1) layered double hydroxides using aqueous hydrogen peroxide: recycle-III

The oxidation reaction of N-methylmorpholine by using ***catalyst A*** which had been used in example 4 was performed in an identical procedure as detailed in example 2, without further addition of fresh catalyst. N-methylmorpholine N-oxide of 94% yield was obtained.

### Example 6

### Oxidation of N-methylmorpholine catalysed by tungstate exchanged with Mg/Al (3:1) layered double hydroxides using aqueous hydrogen peroxide: recycle-IV

The oxidation reaction of N-methylmorpholine by using ***catalyst A*** which had been used in example 5 was performed in an identical procedure as detailed in example 2, without further addition of fresh catalyst. N-methylmorpholine N-oxide of 94% yield was obtained.

### Example 7

### Oxidation of N-methylmorpholine catalysed by tungstate exchanged with Mg/Al (3:1) layered double hydroxides using aqueous hydrogen peroxide: recycle-V

The oxidation reaction of N-methylmorpholine by using ***catalyst A*** which had been used in reaction 6 in an identical procedure as detailed in example 2, without further addition of fresh catalyst. N-methylmorpholine N-oxide of 95% yield was obtained.

### Example 8

### Oxidation of N-methylmorpholine catalysed by molybdate exchanged with Mg/Al (3:1) layered double hydroxides using aqueous hydrogen peroxide

The oxidation reaction of N-methylmorpholine was performed using ***catalyst B*** in an identical procedure as detailed in Example 2. The time taken for the completion of reaction was 3.5 hours. N-methylmorpholine N-oxide of 90% yield was obtained.

### Example 9

### Oxidation of N-methylmorpholine catalysed by vanadate exchanged with Mg/Al (3:1) layered double hydroxides using aqueous hydrogen peroxide

The oxidation reaction of N-methylmorpholine was performed using ***catalyst C*** in an identical procedure as detailed in Example 2. The time taken for the completion of reaction was 3.5 hours. N-methylmorpholine N-oxide of 40% yield was obtained.

### Example 10

### Oxidation of N-methylmorpholine catalysed by tungstate exchanged with Mg Al (3:1) layered double hydroxides using aqueous hydrogen peroxide

The oxidation reaction of N-methylmorpholine was performed using ***catalyst D*** in an identical procedure as detailed in Example 2. N-methylmorpholine N-oxide of 95% yield was obtained.

### Example 11

### Oxidation of N-methylmorpholine catalysed by tungstate exchanged with Mg/Al (3:1) layered double hydroxides using aqueous hydrogen peroxide

The oxidation reaction of N-methylmorpholine was performed using ***catalyst E*** in an identical procedure as detailed in Example 2. N-methylmorpholine N-oxide of 96% yield was obtained.

### Example 12

### Oxidation of N-methylmorpholine catalysed by tungstate exchanged with Mg/Al (3:1) layered double hydroxides using aqueous hydrogen peroxide

The oxidation reaction of N-methylmorpholine was performed using ***catalyst F*** in an identical procedure as detailed in Example 2. The time taken for the completion of reaction was 1.5 hours. N-methylmorpholine N-oxide of 95% yield was obtained.

### Example 13

### Oxidation of N-methylmorpholine catalysed by tungstate exchanged with Mg/Al (3:1) layered double hydroxides using aqueous hydrogen peroxide

The oxidation reaction of N-methylmorpholine was performed using ***catalyst G*** in an identical procedure as detailed in Example 2. The time taken for the completion of reaction was 1.5 hours. N-methylmorpholine N-oxide of 95% yield was obtained.

### Example 14

### Oxidation of N-methylmorpholine catalysed by LDH-{[PO₄WO(O₂)]₄}:

The oxidation reaction of N-methylmorpholine was performed using ***catalyst H*** in an identical procedure as detailed in Example 2. The time taken for the completion of reaction was 3.5 hours. N-methylmorpholine N-oxide of 40% yield was obtained.

### Example 15

### Oxidation of N-methylmorpholine catalysed by Na₂WO₄

The oxidation reaction of N-methylmorpholine was performed using Na₂WO₄ in an identical procedure as detailed in Example 2. The time taken for the completion of reaction was 3.5 hours. N-methylmorpholine N-oxide of 75% yield was obtained.

### Example 16

### Oxidation of N-methylmorpholine catalysed by Na₂VO₃

The oxidation reaction of N-methylmorpholine was performed using Na₂VO₃ in an identical procedure as detailed in Example 2. The time taken for the completion of reaction was 3.5 hours. N-methylmorpholine N-oxide of 15% yield was obtained.

### Example 17

### Oxidation of N-methylmorpholine catalysed by Na₂MoO₄

The oxidation reaction of N-methylmorpholine was performed using Na₂MoO₄ in an identical procedure as detailed in Example 2. The time taken for the completion of reaction was 3.5 hours. N-methylmorpholine N-oxide of 48% yield was obtained.

### Example 18

### Oxidation of N-methylmorpholine catalysed by tungstate exchanged with Mg/Al (3:1) layered double hydroxides using aqueous hydrogen peroxide

The four-necked flask was charged with 0.22ml (2mmol) of N-methylmorpholine, 200mg of ***catalyst A*** and 50ml of water. To this 6mg of dodecylbenzenesulfonic acid sodium salt was added as surfactant. To the mixture was added dropwise 6.6ml (6 mmol) of a 30% by weight of aqueous solution of hydrogen peroxide for period of 0.5 hours in 2 to 3 portions at 25°C under continuous stirring. Continued the reaction for another 0.5hour. After the completion of the reaction (followed by TLC), the catalyst was filtered off and washed with methanol. To the filtrate a small amount of manganese dioxide was added to decompose the unreacted hydrogen peroxide. The treated reaction mixture was filtered to remove the solid MnO₂ and concentrated under reduced pressure to obtain the product. The product thus obtained was purified by column chromatography to afford the corresponding amine oxide. N-methylmorpholine N-oxide of 96% yield was obtained. This product is commercially available from Fluka, Aldrich, Lancaster and Merck companies.

### Example 19

### Oxidation of triethyl amine catalysed by tungstate exchanged with Mg/Al (3:1) layered double hydroxides using aqueous hydrogen peroxide

The oxidation reaction of triethyl amine by using ***catalyst A*** was performed in an identical procedure as detailed in example 2. The time taken for the completion of reaction was 3 hours. Triethyl amine N-oxide of 96% yield was obtained.

### Example 20

### Oxidation of triethyl amine catalysed by tungstate exchanged with Mg/Al (3:1) layered double hydroxides using aqueous hydrogen peroxide

The oxidation reaction of triethyl amine by using ***catalyst A*** was performed in an identical procedure as detailed in example 18. The time taken for the completion of reaction was 1.5 hours. Triethyl amine N-oxide of 96% yield was obtained.

### Example 21

### Oxidation of tributyl amine catalysed by tungstate exchanged with Mg/Al (3:1) layered double hydroxides using aqueous hydrogen peroxide

The oxidation reaction of tributyl amine was performed by using ***catalyst A*** in an identical procedure as detailed in example 2. The time taken for the completion of reaction was 3 hours. Tributyl amine N-Oxide of 94% yield was obtained.

### Example 22

### Oxidation of N,N-dibutyl benzylamine catalysed by tungstate exchanged with Mg/Al (3:1) layered double hydroxides using aqueous hydrogen peroxide

The oxidation reaction of N,N-dibutyl benzylamine was performed by using ***catalyst A*** in an identical procedure as detailed in example 2. The time taken for the completion of reaction was 1.5 hours. N, N-dibutyl benzyl amine N-oxide of 96% yield was obtained.

### Example 23

### Oxidation of N,N-dibutyl benzylamine catalysed by tungstate exchanged with Mg/Al (3:1) layered double hydroxides using aqueous hydrogen peroxide

The oxidation reaction of N,N-dibutyl benzylamine was performed by using ***catalyst A*** in an identical procedure as detailed in example 18. N, N-dibutyl benzyl amine N-oxide of 95% yield was obtained.

### Example 24

### Oxidation of N-benzyl piperidine catalysed by tungstate exchanged with Mg Al (3:1) layered double hydroxides using aqueous hydrogen peroxide

The oxidation reaction of N-benzyl piperidine was performed by using ***catalyst A,*** in an identical procedure as detailed in example 2. The time taken for the completion of reaction was 3 hours. N-benzyl piperidine N-oxide of 97% yield was obtained.

### Example 25

### Oxidation of N-benzyl piperidine catalysed by tungstate exchanged with Mg/Al (3:1) layered double hydroxides using aqueous hydrogen peroxide

The oxidation reaction of N-benzyl piperidine was performed by using ***catalyst A,*** in an identical procedure as detailed in example 18. N-benzyl piperidine N-oxide of 97% yield was obtained.

### Example 26

### Oxidation of N,N-dimethyldecylamine catalysed by tungstate exchanged with Mg/Al (3:1) layered double hydroxides using aqueous hydrogen peroxide

The oxidation reaction of N,N-dimethyldecylamine was performed by using ***catalyst A*** in an identical procedure as detailed in example 2. The time taken for the completion of reaction was 2.5 hours. N, N-dimethyldecylamine N-oxide of 97% yield was obtained. This product is commercially available from Lonza Inc., With trade name Barlox 10S (Specification 30 weight percent decyldimethyl tertiary amine oxide).

### Example 27

### Oxidation of N,N-dimethyloctylamine catalysed by tungstate exchanged with Mg/Al (3:1) layered double hydroxides using aqueous hydrogen peroxide

The oxidation reaction of N,N-dimethyloctylamine was performed by using ***catalyst A*** in an identical procedure as detailed in example 2. The time taken for the completion of reaction was 2.5 hours. N,N-dimethyloctylamine N-oxide of 95% yield was obtained.

### Example 28

### Oxidation of N,N-dimethyloctylamine catalysed by tungstate exchanged with Mg/Al (3:1) layered double hydroxides using aqueous hydrogen peroxide

The oxidation reaction of N,N-dimethyloctylamine was performed by using ***catalyst A*** in an identical procedure as detailed in example 18. N,N-dimethyloctylamine N-oxide of 95% yield was obtained.

### Example 29

### Oxidation of N,N-dimethyl benzylamine catalysed by tungstate exchanged with Mg/Al (3:1) layered double hydroxides using aqueous hydrogen peroxide

The oxidation reaction of N,N-dimethyl benzylamine was performed by using ***catalyst A*** in an identical procedure as in Example 2. The time taken for the completion of reaction was 1.5 hours. N,N-dimethyl benzylamine amine N-oxide of 95% yield was obtained

### Example 30

### Oxidation of N,N-dimethyl benzylamine catalysed by tungstate exchanged with Mg/Al (3:1) layered double hydroxides using aqueous hydrogen peroxide

The oxidation reaction of N,N-dimethyl benzylamine was performed by using ***catalyst A*** in an identical procedure as in example 18. N,N-dimethyl benzylamine amine N-oxide of 96% yield was obtained

### Example 31

### Oxidation of N,N-dimethylcyclohexylamine catalysed by tungstate exchanged with Mg/Al (3:1) layered double hydroxides using aqueous hydrogen peroxide

The oxidation reaction of N,N-dimethylcyclohexylamine by using ***catalyst A*** was performed in an identical procedure as detailed in example 2. The time taken for the completion of reaction was 3 hours. N,N-dimethylcyclohexylamine N-oxide of 96% yield was obtained.

### Example 32

### Oxidation of N,N-dimethylcyclohexylamine catalysed by tungstate exchanged with Mg/Al (3:1) layered double hydroxides using aqueous hydrogen peroxide

The oxidation reaction of N,N-dimethylcyclohexylamine by using ***catalyst A*** was performed in an identical procedure as detailed in example 18. N,N-dimethylcyclohexylamine N-oxide of 95% yield was obtained.

### Example 33

### Oxidation of dibutyl amine catalysed by tungstate exchanged with Mg/Al (3:1) layered double hydroxides using aqueous hydrogen peroxide

The oxidation reaction of dibutyl amine was performed by using ***catalyst D*** in an identical procedure as detailed in example 2. The time taken for the completion of reaction was 3 hours. N-butylidene-butylamine N-oxide of 96% yield was obtained.

### Example 34

### Oxidation of dibutyl amine catalysed by tungstate exchanged with Mg/Al (3:1) layered double hydroxides using aqueous hydrogen peroxide

The oxidation reaction of dibutyl amine was performed by using ***catalyst E*** in an identical procedure as detailed in example 2. The time taken for the completion of reaction was 3 hours. N-butylidene-butylamine N-oxide of 95% yield was obtained.

### Example 35

### Oxidation of dibutyl amine catalysed by tungstate exchanged with Ni/Al (3:1) layered double hydroxides using aqueous hydrogen peroxide

The oxidation reaction of dibutyl amine was performed by using ***catalyst F*** in an identical procedure as detailed in example 2. The time taken for the completion of reaction was 3 hours. N-butylidene-butylamine N-oxide of 96% yield was obtained.

### Example 36

### Oxidation of dibutyl amine catalysed by tungstate exchanged with Ni/Al (3:1) layered double hydroxides using aqueous hydrogen peroxide

The oxidation reaction of dibutyl amine was performed by using ***catalyst G*** in an identical procedure as detailed in example 2. The time taken for the completion of reaction was 3 hours. N-butylidene-butylamine N-oxide of 95% yield was obtained

### Example 37

### Oxidation of dibutyl amine catalysed by tungstate exchanged with Mg/Al (3:1) layered double hydroxides using aqueous hydrogen peroxide

The oxidation reaction of dibutyl amine was performed by using ***catalyst A*** in an identical procedure as detailed in example 2. The time taken for the completion of reaction was 3 hours. N-butylidene-butylamine N-oxide of 97% yield was obtained.

### Example 38

### Oxidation of dibenzyl amine catalysed by tungstate exchanged with Mg/Al (3:1) layered double hydroxides using aqueous hydrogen peroxide

The oxidation reaction of dibenzyl amine was performed by using of ***catalyst A*** in an identical procedure as detailed in example 2. The time taken for the completion of reaction was 5 hours. N-benzylidenebenzylamine N-oxide of 60% yield was obtained.

### Example 39

### Oxidation N-benzyl phenethylamine catalysed by tungstate exchanged with Mg/Al (3:1) layered double hydroxides using aqueous hydrogen peroxide

The oxidation reaction of N-benzyl phenethylamine was performed by using ***catalyst A*** in an identical procedure as in example 2. The time taken for the completion of reaction was 6 hours. N-(1-methyl benzylidine) benzylamine N-oxide of 90% yield was obtained.

### Example 40

### Oxidation of N-phenyl benzylamine amine catalysed by tungstate exchanged with Mg/Al (3:1) layered double hydroxides using aqueous hydrogen peroxide

The reaction oxidation reaction of N-phenyl benzytamine was performed by using ***catalyst A*** in an identical procedure as detailed in example 2. The time taken for the completion of reaction was 4 hours. N-bezylidine phenylamine N-oxide of 93% yield was obtained.

### Example 41

### Oxidation piperidine catalysed by tungstate exchanged with Mg/Al (3:1) layered double hydroxides using aqueous hydrogen peroxide

The ox idation reaction of piperidine by using ***catalyst A*** was performed in an identical procedure as detailed in example 2. The time taken for completion of reaction was 3 hours. 2,3,4,5 tetrahydro pyridine N-oxide of 92% yield was obtained

### Example 42

### Oxidation of 1,2,3,4-tetrahydroisoquinoline catalysed by tungstate exchanged with Mg/Al (3:1) layered double hydroxides using aqueous hydrogen peroxide

The oxidation reaction of 1,2,3,4-tetrahydroisoquinoline performed by using ***catalyst A*** in an identical procedure as detailed in example 2. The time taken for completion of reaction was 5 hours. 3,4 dihydroisoquinoline N-oxide of 93% yield was obtained.

### Example 43

### Oxidation of diisopropyl amine catalysed by tungstate exchanged with Mg/Al (3:1) layered double hydroxides using aqueous hydrogen peroxide

The reaction oxidation reaction of diisopropyl amine was performed by using ***catalyst A*** in an identical procedure as detailed in example 2. The time taken for the completion of reaction was 3 hours. N-(1-methylethylidine)-1-methylethylamine N-oxide of 92% yield was obtained.

**Table 1.**

| Reusability of the catalyst in the oxidation of N-methylmorpholine catalysed by tungstate exchanged with Mg/Al (3:1) layered double hydroxides **(*Catalyst A*)** using aqueous hydrogen peroxide^{a} | | | | | |
|---|---|---|---|---|---|
| Ex. No | Tertiary amine | Cycle | Time(h) | Amine oxide | Yield^{b} |
| 2 | N-methylmorpholine | 1 | 1.0 | N-methylmorpholine N-oxide | 96 |
| 3 | N-methylmorpholine | 2 | 1.0 | N-methylmorpholine N-oxide | 95 |
| 4 | N-methylmorpholine | 3 | 1.0 | N-methylmorpholine N-oxide | 94 |
| 5 | N-methylmorpholine | 4 | 1.0 | N-methylmorpholine N-oxide | 94 |
| 6 | N-methylmorpholine | 5 | 1.0 | N-methylmorpholine N-oxide | 94 |
| 7 | N-methylmorpholine | 6 | 1.0 | N-methylmorpholine N-oxide | 95 |

| | | | | | |
|---|---|---|---|---|---|
| ^{a} Reaction conditions as exemplified in example 2 | | | | | |
| ^{b} Isolated yields | | | | | |

**Table 2.**

| The catalytic N-oxidation of N-methylmorpholine to N-methylmorpholine N-oxide in water using various metal ion-exchanged LDH catalysts and their homogeneous analogues^{a} | | | |
|---|---|---|---|
| Ex. No | Catalyst | Time(h) | Yield^{c} |
| 8 | LDH-MoO₄²⁻ **(Catalyst B)** | 3.5 | 90 |
| 9 | LDH-VO₃⁻ **(Catalyst C)** | 3.5 | 40 |
| 10 | LDH-WO₄²⁻ **(Catalyst D)** | 1.0 | 95 |
| 11 | LDH-WO₄²⁻ **(Catalyst E)** | 1.0 | 96 |
| 12 | Ni-Al-LDH- WO₄²⁻ **(Catalyst F)** | 1.5 | 95 |
| 13 | Ni-Al-LDH- WO₄²⁻ **(Catalyst G)** | 1.5 | 96 |
| 14 | LDH-{PO₄WO(O₂)]₄} **(Catalyst H)** | 3.5 | 40 |
| 15 | Na₂WO₄ | 3.5 | 75 |
| 16 | NaVO₃ | 3.5 | 15 |
| 17 | Na₂MoO₄ | 3.5 | 48 |

| | | | |
|---|---|---|---|
| ^{a} Reaction conditions as exemplified in example 2 | | | |
| ^{b} Isolated yields | | | |

**Table 3.**

| N-oxidation of aliphatic *tert*-amines catalysed by LDH-WO₄²⁻ **(*Catalyst A*)** in water^{a} (procedure **I**) or water in combination of dodecylbenzenesufonicacid sodium salt^{b} (procedure **II**). | | | | | |
|---|---|---|---|---|---|
| Ex. No | Tertiary amine | Procedure | Amine oxide | Time(h) | Yield^{c} |
| 18 | N-methyl morpholine | **II** | N-methylmorpholine N-oxide | 1.0 | 96 |
| 19 | Triethyl amine | **I** | Triethyl amine N-oxide | 3.0 | 96 |
| 20 | Triethyl amine | **II** | Triethyl amine N-oxide | 1.5 | 96 |
| 21 | Tributyl amine | **I** | Tributyl amine N-oxide | 3.0 | 94 |
| 22 | N,N-dibutyl benzyl amine | **I** | N,N-dibutyl benzyl amine N-oxide | 1.5 | 96 |
| 23 | N,N-dibutyl benzyl amine | **II** | N,N-dibutyl benzyl amine N-oxide | 1.0 | 95 |
| 24 | N-benzyl piperidine | **I** | N-benzyl piperidine N-oxide | 3.0 | 97 |
| 25 | N-benzyl piperidine | **II** | N-benzyl piperidine N-oxide | 1.0 | 97 |
| 26 | N,N-dimethyl decyl amine | **I** | N,N-dimethyl decyl amine N-oxide | 2.5 | 97 |
| 27 | N,N-dimethyl octyl amine | **I** | N,N-dimethyl octyl amine N-oxide | 2.5 | 95 |
| 28 | N,N-dimethyl octyl amine | **II** | N,N-dimethyl octyl amine N-oxide | 1.0 | 95 |
| 29 | N,N-dimethyl benzyl amine | **I** | N,N-dimethyl benzyl amine | 1.5 | 95 |
| 30 | N,N-dimethyl benzyl amine | **II** | N-oxide N,N-dimethyl benzyl amine N-oxide | 1.0 | 96 |
| 31 | N,N-dimethyl cyclohexylamine | **I** | N,N-dimethyl yclohexylamine N-oxide | 3.0 | 96 |
| 32 | N,N-dimethyl cyclohexylamine | **II** | N,N-dimethyl yclohexylamine N-oxide | 1.0 | 95 |

| | | | | | |
|---|---|---|---|---|---|
| ^{a} Reaction conditions as exemplified in example 2 | | | | | |
| ^{b} Reaction conditions as exemplified in example 18 | | | | | |
| ^{b} Isolated yields | | | | | |

**Table 4.**

| Oxidation of secondary amines catalysed by anion of transition metal oxides exchanged layered double hydroxides using aqueous hydrogen peroxide | | | | | |
|---|---|---|---|---|---|
| Ex.No | Secondary amine | Catalyst | Amine oxide (nitrone) | Time(h) | Yield ^{b} |
| 33 | *Dibutyl amine* | ***D*** | N-butylidene-butylamine N-oxide | 3 | 96 |
| 34 | Dibutyl amine | **E** | N-butylidene-butylamine N-oxide | 3 | 95 |
| 35 | Dibutyl amine | **F** | N-butylidene-butylamine N-oxide | 3 | 96 |
| 36 | Dibutyl amine | **G** | N-butylidene-butylamine N-oxide | 3 | 95 |
| 37 | Dibutyl amine | **A** | N-butylidene-butylamine N-oxide | 3 | 97 |
| 38 | Dibenzyl amine | **A** | N-benzylidene benzylamine N-oxide | 5 | 60 |
| 39 | N-benzyl phenethyl amine | **A** | N-(1-methylbenzylidene) benzylamine N-oxide | 6 | 90 |
| 40 | N-Phenyl benzyl amine | **A** | N-benzylidene phenylamine N-oxide | 4 | 93 |
| 41 | Piperidine | **A** | 2,3,4,5 Tetrahydro pyridine N-oxide | 3 | 92 |
| 42 | 1,2,3,4 Tetrahydro isoquinoline | **A** | 3,4, Dihydroisoquinoline N-oxide | 5 | 93 |
| 43 | Diisopropyl amine | **A** | N-(1-methylethylidene)1-methylethyl amine N-oxide | 3 | 92 |

| | | | | | |
|---|---|---|---|---|---|
| ^{a} Reaction conditions as exemplified in example 2 ^{b} Isolated yields | | | | | |

The main advantages of the present invention are:
1. The present process is eco-friendly and simple.
2. The catalyst is cheap, non-corrosive, recyclable for several times and heterogeneous in nature.
3. The reactions are conducted in water, an eco-friendly benign solvent.
4. The reaction conditions are very mild, being the reaction temperature ranges between 10-25°C.
5. The hydrogen peroxide used is 30% by weight, which is more environmentally friendly than some of the harsh reagents used in the prior art.
6. The process is economical and is accomplished in short time with high productivity.
7. The amount of effluent formed in this process is minimized because the catalyst and solvent are recovered/recycled and reused.
8. The process provides high quality of the product without resulting in gel formation, during the course of reaction.

## Claims

1. A process for the preparation of amine oxide which comprises reacting a tertiary or secondary amine with hydrogen peroxide as an oxidant in presence of a recyclable heterogeneous layered double hydroxide exchanged with one of the anions of transition metal oxides as a catalyst in a solvent selected from the group consisting of water or water containing dodecylbenzenesulfonic acid sodium salt as additive, at a temperature ranging between 10-25°C for a period up to 6 hours under continuous stirring and separating the product by simple filtration and subsequent evaporation of solvents.

2. A process as claimed in claim 1 wherein the heterogeneous catalyst used is layered double hydroxide (LDH) exchanged with transition metal oxides selected from a group consisting of tungstate, molybdate, vanadate and their polyanions as polyoxometalates having formula I: [M^{II}₍₁₋ₓ₎M^{III}ₓ(OH)₂][Mⁿ⁻]ₓ₂.zH₂O, which is derived from LDH having formula II [M^{II}₍₁₋ₓ₎M^{III}ₓ(OH)₂][Aⁿ⁻]ₓ₂.zH₂O where Mⁿ is an anion of transition metal oxide selected from a group consisting of W, Mo, V and Aⁿ⁻ is an interstitial anion, selected from nitrate, chloride and M^{II} is a divalent cation selected from the group consisting of Mg²⁺, Mn²⁺, Fe²⁺, V²⁺, Co²⁺, Ni²⁺, Cu²⁺, Zn²⁺ Pd²⁺, or Ca²⁺ and M^{III} is a trivalent ion selected from the group consisting of Al³⁺, Cr³⁺, V³⁺, Mn³⁺, Fe³⁺, Co³⁺, Ni³⁺, Rh³⁺, Ru³⁺, Ga³⁺ or La³⁺, x is the mole fraction having a value ranging from 0.2 to 0.33 and z is the number of water molecules ranging from 1 to 4.

3. A process as claimed in claim 1 wherein the tertiary amines, have the general formula R¹R²NR³ wherein R¹, R² and R³ are the same or different and are straight-chain or branched-chain groups selected from alkyl, alkenyl and aralkyls having C₁-C₂₄ carbons including dimethyl decyl amine, dimethyl dodecyl amine, dimethylbenzylamine triethylamine, tributylamine and cyclic amines selected from imidazolines, pyridines, N-substituted piperazines, N-substituted piperadines or N-substituted morpholines such as N-methylmorpholine.

4. A process as claimed in claim 1 where in the secondary amines used in the system have the general formula R¹R²NH wherein R¹ and R² may be the same or different and are straight-chain or branched chain groups selected from alkyl, alkenyl and aralkyl having C₁-C₂₄ carbons including dibutyl amine, dibenzyl amine, N-benzyl phenethylamine, N-phenyl benzylamine, cyclic amines selected from piperidine, 1,2,3,4 tetrahydro isoquinoline.

5. A process as claimed in claim 1 wherein up to 30% by weight of aqueous hydrogen peroxide is added slowly in a controlled manner during a period of up to 2 hours.

6. A process as claimed in claim 1 wherein the catalyst introduced in the system is 6-12 weight % anion of transition metal oxides selected from tungstate, molybdate, vanadate and their polyanions as polyoxometalates.

7. A process as claimed in claim 1 wherein the amount of hydrogen peroxide used ranges between 2 to 6 moles per mole of secondary or tertiary amine.

## Patentansprüche

1. Verfahren zur Herstellung von Aminoxiden, umfassend das Umsetzen eines tertiären oder sekundären Amins mit Wasserstoffperoxid als Oxidationsmittel in Gegenwart eines recycelbaren, heterogenen schichtförmigen Doppelhydroxids, das mit einem der Anionen von Übergangsmetalloxiden ausgetauscht ist, als Katalysator, in einem Lösungsmittel, das aus der Gruppe ausgewählt ist, die aus Wasser oder Wasser, das Dodecylbenzolsulfonsäure-Natriumsalz als Additiv enthält, besteht, bei einer Temperatur im Bereich zwischen 10-25°C für einen Zeitraum bis zu 6 Stunden unter kontinuierlichem Rühren, und Abtrennen des Produkts durch einfache Filtration und anschließende Verdampfung der Lösungsmittel.

2. Verfahren nach Anspruch 1, wobei der verwendete heterogene Katalysator ein schichtförmiges Doppelhydroxid (LDH) ist, das mit Übergangsmetalloxiden ausgetauscht ist, die aus der Gruppe ausgewählt sind, die aus Wolframat, Molybdat, Vanadat und deren Polyanionen als Polyoxometalaten mit der Formel I besteht: [ M^{II}₍₁₋ₓ₎M^{III}ₓ(OH)₂][Mⁿ⁻]_{x/2}·zH₂O, die aus LDH mit der Formel II [ M^{II}₍₁₋ₓ₎M^{III}ₓ(OH)₂][Aⁿ⁻]_{x/2}·zH₂O abgeleitet ist/sind, wobei Mⁿ⁻ ein Anion eines Übergangsmetalloxids ist, das aus der Gruppe ausgewählt ist, die aus W, Mo und V besteht, und Aⁿ⁻ ein interstitielles Anion ist, das aus Nitrat und Chlorid ausgewählt ist, und M^{II} ein zweiwertiges Kation ist, das aus der Gruppe ausgewählt ist, die aus Mg²⁺, Mn²⁺, Fe²⁺, V²⁺, Co²⁺, Ni²⁺, Cu²⁺, Zn²⁺, Pd²⁺, oder Ca²⁺ besteht, und M^{III} ein dreiwertiges Ion ist, das aus der Gruppe ausgewählt ist, die aus Al³⁺, Cr³⁺, V³⁺, Mn³⁺, Fe³⁺, Co³⁺, Ni³⁺, Rh³⁺, Ru³⁺, Ga³⁺ oder La³⁺ besteht, x der Molenbruch mit einem Wert im Bereich von 0,2 bis 0,33 ist und z die Anzahl der Wassermoleküle ist, die von 1 bis 4 reicht.

3. Verfahren nach Anspruch 1, wobei die tertiären Amine die allgemeine Formel R¹R²NR³ aufweisen, wobei R¹, R² und R³ gleich oder verschieden sind und geradkettige oder verzweigtkettige Gruppen sind, die aus Alkyl, Alkenyl und Aralkyl mit C₁-C₂₄ Kohlenstoffen ausgewählt sind, einschließlich Dimethyldecylamin, Dimethyldodecylamin, Dimethylbenzylamin, Triethylamin, Tributylamin und cyclischen Aminen, ausgewählt aus Imidazolinen, Pyridinen, N-substituierten Piperazinen, N-substituierten Piperadinen oder N-substituierten Morpholinen, wie N-Methylmorpholin.

4. Verfahren nach Anspruch 1, wobei die in dem System verwendeten sekundären Amine die allgemeine Formel R¹R²NH aufweisen, worin R¹ und R² gleich oder verschieden sein können und geradkettige oder verzweigtkettige Gruppen sind, ausgewählt aus Alkyl, Alkenyl und Aralkyl mit C₁-C₂₄ Kohlenstoffen, einschließlich Dibutylamin, Dibenzylamin, N-Benzylphenethylamin, N-Phenylbenzylamin und cyclischen Aminen, ausgewählt aus Piperidin und 1,2,3,4-Tetrahydroisochinolin.

5. Verfahren nach Anspruch 1, wobei bis zu 30 Gew.-% wäßriges Wasserstoffperoxid langsam auf kontrollierte Weise während eines Zeitraums von bis zu 2 Stunden zugegeben werden.

6. Verfahren nach Anspruch 1, wobei der in das System eingeführte Katalysator 6-12 Gew.-% des Anions der Übergangsmetalloxide, ausgewählt aus Wolframat, Molybdat, Vanadat und deren Polyanionen als Polyoxometallaten, beträgt.

7. Verfahren nach Anspruch 1, wobei der Anteil des verwendeten Wasserstoffperoxids im Bereich von 2 bis 6 Mol pro Mol des sekundären oder tertiären Amins reicht.

## Revendications

1. Un procédé de préparation d'oxydes d'amine qui comprend la réaction d'une amine tertiaire ou secondaire avec du peroxyde d'hydrogène en tant qu'oxydant en présence d'un hydroxyde double lamellaire hétérogène recyclable échangé avec un des anions d'oxydes de métaux de transition utilisé en tant que catalyseur dans un solvant choisi dans le groupe consistant en l'eau ou l'eau contenant le sel sodique de l'acide dodécylbenzènesulfonique en tant qu'additif, à une température comprise dans la plage allant de 10 à 25 °C, pendant une durée allant jusqu'à 6 heures, sous agitation continue et la séparation du produit par filtration simple et évaporation ultérieure des solvants.

2. Un procédé selon la revendication 1, dans lequel le catalyseur hétérogène utilisé est un hydroxyde double lamellaire (LDH) échangé avec des oxydes de métaux de transition choisis dans le groupe consistant en les tungstate, molybdate, vanadate et leurs polyanions en tant que polyoxométallates, ayant la formule I: [M^{II}₍₁₋ₓ₎M^{III}x(OH)₂][Mⁿ⁻]_{x/2}.zH₂O, dérivé du LDH ayant la formule II [M^{II}₍₁₋ₓ₎M^{III}x(OH)₂][Aⁿ⁻]_{x/2}.zH₂O, où Mⁿ⁻ représente un anion d'un oxyde de métaux de transition choisi dans le groupe constitué de W, Mo, V, et Aⁿ⁻ représente un anion interstitiel, choisi parmi le nitrate et le chlorure et M^{II} représente un cation bivalent choisi dans le groupe consistant en Mg²⁺, Mn²⁺, Fe²⁺, V²⁺, Co²⁺, Ni²⁺, Cu²⁺, Zn²⁺, Pd²⁺ ou Ca²⁺, et M^{III} représente un ion trivalent choisi dans le groupe consistant Al³⁺, Cr³⁺, V³⁺, Mn³⁺, Fe³⁺, Co³⁺, Ni³⁺, Rh³⁺, Ru³⁺, Ga³⁺ ou La³⁺, x est la fraction molaire dont la valeur est comprise dans la plage allant de 0,2 à 0,33 et z est le nombre de molécules d'eau compris entre 1 et 4.

3. Un procédé selon la revendication 1, dans lequel les amines tertiaires ont la formule générale R¹R²NR³, dans laquelle R¹, R² et R³, qui peuvent être identiques ou différents, sont des groupes à chaîne linéaire ou ramifiée choisis parmi les groupes alkyle, alcényle et aralkyle ayant de 1 à 24 atomes de carbone, comprenant la diméthyldécylamine, la diméthyldodécylamine, la diméthylbenzylamine, la triéthylamine, la tributylamine, et des amines cycliques choisies parmi les imidazolines, les pyridines, les pipérazines N-substituées, les pipéridines N-substituées ou les morpholines N-substituées, telles que la N-méthylmorpholine.

4. Un procédé selon la revendication 1, dans lequel les amines secondaires utilisées dans le système ont la formule générale R¹R²NH, dans laquelle R¹ et R² peuvent être identiques ou différents et sont des groupes à chaîne linéaire ou ramifiée choisis parmi les groupes alkyle, alcényle et aralkyle ayant de 1 à 24 atomes de carbone, comprenant la dibutylamine, la dibenzylamine, la N-benzylphénéthylamine, la N-phénylbenzylamine et les amines cycliques choisies parmi la pipéridine, la 1,2,3,4-tétrahydroisoquinoléine.

5. Un procédé selon la revendication 1, dans lequel on ajoute lentement du peroxyde d'hydrogène aqueux jusqu'à 30 % en poids de façon contrôlée pendant une période de temps allant jusqu'à 2 heures.

6. Un procédé selon la revendication 1, dans lequel le catalyseur introduit dans le système comprend de 6 à 12 % en poids d'anions d'oxydes de métaux de transition choisis parmi le tungstate, le molybdate, le vanadate et leurs polyanions sous la forme de polyoxométallates.

7. Un procédé selon la revendication 1, dans lequel la quantité de peroxyde d'hydrogène utilisé est comprise dans la plage allant de 2 à 6 moles par mole d'amine secondaire ou tertiaire.
